# EUROPÄISCHE PATENTSCHRIFT

(11) **EP 3 077 757 B1**
(45) Veröffentlichungstag und Bekanntmachung des Hinweises auf die Patenterteilung: **18.04.2018**
(21) Anmeldenummer: 14825251.3
(22) Anmeldetag: 28.11.2014
(51) Int. Cl.: F41A 17/06

(54) **SCHUSSWAFFE MIT SICHERUNGSSYSTEM**
WEAPON WITH SAFETY SYSTEM
ARME À FEU AVEC SYSTÈME DE SÉCURITÉ

(30) Priorität: 05.12.2013 AT 508062013
(43) Veröffentlichungstag der Anmeldung: 12.10.2016
(73) Patentinhaber: Neuhold, Bernhard, 8753 Fohnsdorf (AT); Gruber, Ernst, 8750 Judenburg (AT)
(72) Erfinder: Neuhold, Bernhard, 8753 Fohnsdorf (AT); Gruber, Ernst, 8750 Judenburg (AT)
(74) Vertreter: Wirnsberger, Gernot
(86) Internationale Anmeldenummer: PCT/AT2014/050284
(87) Internationale Veröffentlichungsnummer: WO 2015/081358

(56) Entgegenhaltungen:
- DE-A1- 4 413 685
- DE-A1- 19 607 927
- US-A- 4 467 545
- US-A1- 2008 282 595
- US-A1- 2013 019 510

## Beschreibung

Die Erfindung betrifft eine Schusswaffe, mit einer auf einen Schlagbolzen wirkenden Schlagfeder, welche spannbar ist, um die Schusswaffe in einen schussbereiten Zustand zu bringen. Eine Schusswaffe der eingangs genannten Art ist aus DE 44 13 685 A1 bekannt. Darüber hinaus betrifft die Erfindung ein Sicherungssystem für eine Schusswaffe. Weiter betrifft die Erfindung ein Verfahren zur Entspannung einer Schusswaffe mit einer auf einen Schlagbolzen wirkenden Schlagfeder, welche spannbar ist.

Darüber hinaus betrifft die Erfindung eine Verwendung einer derartigen Schusswaffe. Aus dem Stand der Technik sind beispielsweise als Gewehre ausgebildete Schusswaffen der eingangs genannten Art bekannt, welche in der Regel zur Jagd eingesetzt werden. Dabei wird das Gewehr normalerweise durch Bewegen eines üblicherweise an einem Schaft angeordneten Spannschiebers von einer ersten Position in eine zweite Position in einen schussbereiten Zustand gebracht, indem durch die Bewegung des Spannschiebers eine Schlagfeder gespannt wird. Bei einer weiteren konstruktiven Ausführung von Gewehren wird die Schlagfeder durch ein Abkippen bzw. Brechen des Laufes gespannt. Um den Spannschieber in der zweiten Position und die Schlagfeder gespannt zu halten, ist meist ein einrastender Mechanismus vorgesehen, welcher üblicherweise eine Sperrklinke umfasst. Wenn die Schlagfeder gespannt und das Gewehr geladen ist, wird bei Betätigen eines Abzuges der Sperrmechanismus gelöst und durch den mit der Schlagfeder verbundenen Schlagbolzen ein Schlag auf eine Patrone ausgeübt, wodurch ein Schuss abgegeben wird.

Bei einem Einsatz eines entsprechenden Gewehres bei der Jagd ist das Gewehr in der Regel geladen und entspannt. Dabei ist eine Schlagfeder entspannt, wodurch das Gewehr in einem sicheren Zustand ist. Weiter kann ein mit einer Sicherung ausgebildetes Gewehr auch bei gespannter Schlagfeder in einen sicheren Zustand gebracht werden, indem eine Sicherung aktiviert wird, welche eine Schussauslösung durch ein Blockieren des Schlagbolzens verhindert. Um eine versehentliche Schussauslösung zu verhindern, wird das Gewehr erst kurz vor Abgabe eines Schusses, wenn ein Ziel bereits identifiziert und anvisiert werden konnte, entsichert bzw. gespannt. Wird nach einer Entsicherung bzw. nach einer Spannung der Schlagfeder kein Schuss abgegeben, wird das Gewehr beispielsweise durch manuelles Bewegen des Spannschiebers von der zweiten Position in die erste Position wieder unter Entspannung der Schlagfeder in einen sicheren Zustand gebracht, bis sich eine neuerliche Gelegenheit zur Schussabgabe bietet.

Es hat sich allerdings gezeigt, dass insbesondere Anfänger häufig darauf vergessen, das Gewehr beispielsweise durch Betätigen des Spannschiebers in einen sicheren Zustand zu bringen. Dies führt bei einem Hantieren mit dem Gewehr, beispielsweise bei einem Positionswechsel des Anfängers, zu einem hohen Verletzungsrisiko, da auch ein unbeabsichtigtes Berühren des Abzuges einen Schuss auslösen kann.

Aus dem Stand der Technik sind verschiedene Vorrichtungen bekannt geworden, um eine Schusswaffe in einen sicheren Zustand zu bringen. Dabei wird üblicherweise der Schlagbolzen durch ein in eine Sicherungsposition bewegtes Sperrelement oder dergleichen blockiert, sodass eine Schussauslösung verhindert ist. Derartige Vorrichtungen werden häufig bei Waffen eingesetzt, welche zu Ausbildungszwecken in Schießständen verwendet werden, um Waffen von Anfängern in einen sicheren Zustand zu bringen. Jedoch sind diese Vorrichtungen für Jagdwaffen ungeeignet, da bei extremen Temperaturen, welche bei einer Jagd im Freien auftreten, das Sperrelement in der Sicherungsposition blockieren kann. Weiter weisen bekannte Vorrichtungen nicht eine für Jagdwaffen, welche über viele Jahre eingesetzt werden, erforderliche robuste Ausführung auf, die auch nach mehreren Jahren ein zuverlässiges automatisches Herstellen eines sicheren Zustandes der Waffe gewährleistet.

Aufgabe der Erfindung ist es daher, eine Schusswaffe der eingangs genannten Art anzugeben, bei welcher ein Verletzungsrisiko durch ein Hantieren mit einer in einem unsicheren Zustand befindlichen Schusswaffe vermieden wird. Des Weiteren soll die Schusswaffe einfach und robust ausgebildet sein, sodass auch bei verschiedenen Außentemperaturen eine Herstellung eines sicheren Zustandes der Schusswaffe nach mehreren Jahren gewährleistet ist.

Weiter soll ein Verfahren der eingangs genannten Art zur Herstellung eines sicheren Zustandes einer Schusswaffe angegeben werden, welches eine zuverlässige Herstellung eines sicheren Zustandes gewährleistet. Gelöst wird die Aufgabe durch eine Schusswaffe nach dem Patentanspruch 1 und ein Verfahren nach dem Patentanspruch 6. Vorteilhafte Ausgestaltungen sind in den Unteransprüchen aufgeführt. Dadurch ist ein Verletzungsrisiko auf konstruktiv einfache Weise vermieden, da ein automatisches Herstellen eines sicheren Zustandes der Schusswaffe durch ein Entspannen der Schlagfeder erfolgt. In der Regel wird bei einem derartigen Entspannen der Schlagfeder auch der Spannschieber von der zweiten Position in die erste Position bewegt. Eine derartige Schusswaffe ist auch robust, weil kein zusätzliches Sperrelement zwischen den Schlagbolzen und die Patrone bewegt werden muss. Somit ist auch bei einer sehr seltenen Auslösung der Betätigungseinrichtung ein Funktionieren derselben gewährleistet, weil die Schusswaffe über die regelmäßig benutzte Schlagfeder in einen sicheren Zustand gebracht wird. Dies ermöglicht auch nach mehreren Jahren, in welchen die Betätigungseinrichtung nicht ausgelöst wurde, ein automatisiertes und zuverlässiges Herstellen eines sicheren Zustandes der Schusswaffe, wenn zumindest eine der vordefinierten Bedingungen eintritt. Als Bedingung, die zu einem Herstellen eines sicheren Zustandes bzw. einem Entspannen der Schusswaffe führt, kann insbesondere ein Überschreiten einer definierten maximalen Bewegung der Schusswaffe aus einer Entsicherungsposition, in welcher die Schusswaffe entsichert wurde, definiert werden. Dadurch kann gewährleistet werden, dass die Schusswaffe immer dann in einen sicheren Zustand gebracht wird, wenn diese aus einer Schussposition bewegt wird, beispielsweise um eine Position eines Schützen zu ändern. Üblicherweise sind hierzu mechanisch und/oder elektrisch wirkende Sensoren vorgesehen. Darüber hinaus kann auch vorgesehen sein, dass die Schusswaffe automatisch in einen sicheren Zustand gebracht wird, wenn eine Kapazität des Energiespeichers einen definierten Schwellwert unterschreitet. Es kann jedoch auch vorgesehen sein, dass ein Unterschreiten des Schwellwertes des Energiespeichers nur eine Warnung auslöst und die Schusswaffe nicht in einen sicheren Zustand gebracht wird. Dadurch ist eine Funktion der Schusswaffe auch bei leerem Energiespeicher gewährleistet.

Eine elektromechanische Betätigungseinrichtung hat sich besonders bewährt, weil diese auf einfache Weise über entsprechende Sensoren ansteuerbar ist, sodass eine einfache Konstruktion mit geringem benötigten Bauraum und wenigen bewegbaren, und somit verschleißanfälligen Teilen erreicht wird. Dadurch ist über einen großen Temperaturbereich eine robuste Auslösung sichergestellt.

Mit Vorteil ist das Sicherungssystem durch ein Herstellen eines unsicheren Zustandes der Schusswaffe aktivierbar und durch ein Herstellen eines sicheren Zustandes der Schusswaffe deaktivierbar. Üblicherweise wird das Sicherungssystem durch ein Spannen der Schlagfeder oder ein Entsichern durch Betätigung einer Sicherung aktiviert. Somit wird auf einfache Weise verhindert, dass der Energiespeicher, üblicherweise ein Akkumulator oder eine Batterie, entladen wird, wenn die Schusswaffe bereits aufgrund einer entspannten Schlagfeder oder einer aktivierten Sicherung in einem sicheren Zustand ist. Dadurch wird eine hohe Lebensdauer des Energiespeichers erreicht. Weil die Schusswaffe normalerweise erst unmittelbar vor einer Schussabgabe in einen unsicheren Zustand gebracht wird, ist das Sicherungssystem in der Regel nur wenige Sekunden aktiviert, in welchen eine Entladung des Energiespeichers erfolgt. Somit muss ein Energiespeicher erst nach mehreren Jahren ausgetauscht oder wiederaufgeladen werden. Weiter ist durch den dadurch langzeitig geladenen Energiespeicher eine dauerhafte Funktion des Sicherungssystems sichergestellt.

Bevorzugt umfasst das Sicherungssystem einen Mikroprozessor zur Betätigung der Betätigungseinrichtung. Dies ermöglicht eine besonders flexible Anpassung bzw. variable Einstellung der Bedingungen, die eine Herstellung eines sicheren Zustandes der Schusswaffe zur Folge haben. Weiter kann mittels des Mikroprozessors auch eine Warnung abgegeben werden, um auf einen unsicheren Zustand hinzuweisen. Hierzu können Mittel zur drahtlosen Kommunikation wie ein Bluetooth-Modul vorgesehen sein, um eine Warnung an ein Mobiltelefon oder einen Pager eines Instruktors zu senden, welcher beispielsweise einen Anfänger auf eine in einem unsicheren Zustand befindliche Schusswaffe hinweisen kann.

Alternativ oder ergänzend kann auch eine Warneinrichtung, insbesondere eine Leuchtdiode, vorgesehen sein, um auf einen unsicheren Zustand der Schusswaffe hinzuweisen. Hierzu ist die Leuchtdiode in der Regel mit einem Mikroprozessor verbunden, durch welchen die Leuchtdiode bei Erkennen eines unsicheren Zustandes und Eintritt einer definierten Bedingung wie ein Ablauf einer Zeitspanne oder eine Bewegung der Schusswaffe um einen definierten Winkel aktiviert wird. Günstig ist es, wenn die Leuchtdiode unterhalb des Spannschiebers und hinter demselben angeordnet ist, da an dieser Position bereits durch eine geringe Leuchtkraft eine gute Wahrnehmung durch den Benutzer erreicht wird. Weiter kann die Leuchtdiode eingesetzt werden, um auf ein Aktivieren der Betätigungseinrichtung bei Herstellung eines unsicheren Zustandes bzw. Spannen der Schusswaffe, einen Energiespeicher mit nur mehr geringer Restladung oder dergleichen hinzuweisen.

Mit Vorteil ist ein mit dem Mikroprozessor verbundener Datenspeicher zur Aufzeichnung von schussrelevanten Daten vorgesehen. Beispielsweise können dadurch auf einfache Weise abgegebene Schüsse und, sofern entsprechende Sensoren wie beispielsweise Bewegungs- oder Beschleunigungssensoren vorgesehen sind, auch Positionen der Schussabgaben oder eingesetzte Munition mitprotokolliert werden. Hierzu kann auch ein GPS-Modul in der Schusswaffe vorgesehen sein. Derartige Daten können auch für eine zustandsorientierte Wartung der Schusswaffe eingesetzt werden.

Zweckmäßigerweise weist das Sicherungssystem zur Herstellung eines sicheren Zustandes der Schusswaffe bei einer vordefinierten Beschleunigung oder einer vordefinierten Beschleunigungsänderung zumindest einen Beschleunigungssensor auf. Dadurch kann die Schusswaffe beispielsweise genau dann gesichert werden, wenn diese aus der Entsicherungsposition um einen bestimmten Winkel verdreht oder gekippt wird. Durch einen oder mehrere Beschleunigungssensoren ist eine Position der Schusswaffe im Raum bestimmbar, indem die auf die einzelnen Beschleunigungssensoren wirkenden Anteile der Erdbeschleunigung gemessen werden. Eine aktuelle Position wird üblicherweise mit der Entsicherungsposition verglichen, um auf unzulässige Bewegungen mit ungesicherter Schusswaffe zu schließen. Beispielsweise kann hierzu im Mikroprozessor ein maximaler Kipp- bzw. Rollwinkel von beispielsweise etwa 30° um eine Längsachse der Schusswaffe definiert werden, sodass eine diesen maximalen Winkel überschreitende Bewegung aus der Entsicherungsposition ein automatisches Herstellen eines sicheren Zustandes bzw. ein Entspannen der Schusswaffe auslöst. Es kann auch vorgesehen sein, dass einem automatischen Herstellen eines sicheren Zustandes eine Warnung vorausgeht, die einen Benutzer auf eine aus einer Schussposition bewegte Schusswaffe hinweist, welche in einem unsicheren Zustand ist. In der Regel sind mehrere Bewegungs- oder Beschleunigungssensoren vorgesehen, um Bewegungen der Schusswaffe in jeder Raumrichtung erfassen sowie eine Position der Schusswaffe besonders genau feststellen zu können. Weiter kann durch Beschleunigungssensoren auch eine Fallerkennung für ungesicherte Schusswaffen umgesetzt werden, durch welchen die Schusswaffe automatisch in einen sicheren Zustand gebracht wird, wenn erkannt wird, dass sich die Schusswaffe in einem freien Fall befindet, bevor diese auf einem Boden aufschlägt. Dadurch können Unfälle durch unbeabsichtigt fallen gelassene oder umfallende Schusswaffen auf einfache Weise verhindert werden.

Zweckmäßigerweise ist zur Herstellung eines sicheren Zustandes der Schusswaffe nach einer vordefinierten Zeit eine Zeiterfassungseinrichtung vorgesehen. Meist ist in einem Mikroprozessor eine elektronische Zeiterfassungseinrichtung integriert, mit welcher nach einer vordefinierten Zeit nach Entsicherung oder Spannen der Schusswaffe eine automatische Herstellung eines sicheren Zustandes der Schusswaffe mittels der Betätigungseinrichtung erfolgen kann. Es kann auch vorgesehen sein, dass nach einer definierten ersten Zeitspanne eine Warnung abgegeben wird und, sofern innerhalb einer definierten zweiten Zeitspanne die Schusswaffe weder gesichert oder entspannt noch ein Schuss abgegeben wird, die Schusswaffe in einen sicheren Zustand gebracht wird. Die Zeitspannen liegen üblicherweise im Bereich von weniger als fünf Minuten, insbesondere fünf Sekunden bis 120 Sekunden.

Mit Vorteil ist insbesondere in einem Schaft oder einem Griffstück der Schusswaffe ein Sensor vorgesehen, mit welchem eine Berührung der Schusswaffe durch einen Benutzer ermittelbar ist. Dadurch ist eine Fallerkennung einfach realisierbar, durch welchen eine in einem unsicheren Zustand befindliche Schusswaffe automatisch in einen sicheren Zustand gebracht werden kann, wenn ein Benutzer die Schusswaffe beispielsweise fallen lässt. Weil ein entsprechendes Ereignis durch den Sensor unmittelbar erkannt wird, kann die Schusswaffe normalerweise in einen sicheren Zustand gebracht werden, bevor diese auf einem Boden aufschlägt. Weiter kann durch den Sensor, welcher beispielsweise als kapazitiver Sensor ausgebildet sein kann, und die mit dem Sensor in Verbindung stehende Betätigungseinrichtung sowie eine elektronische Uhr die Schusswaffe auch dann automatisch in einen sicheren Zustand gebracht werden, wenn ein Benutzer die Schusswaffe eine definierte Zeit nicht berührt hat. Dadurch kann beispielsweise eine ordnungsgemäß abgestellte oder abgelegte Schusswaffe in einen sicheren Zustand gebracht werden, bei welcher auf ein Sichern oder ein Entspannen vergessen wurde.

Zweckmäßigerweise ist die Betätigungseinrichtung zur Ausführung eines Schlages auf eine den Spannschieber in der zweiten Position fixierende Sperrklinke ausgebildet. Dazu ist die Betätigungseinrichtung derart relativ zur Sperrklinke in der Schusswaffe positioniert, dass die Sperrklinke durch die Betätigungseinrichtung betätigbar ist.

Günstig ist es, wenn die Betätigungseinrichtung derart relativ zur Sperrklinke angeordnet ist, dass der Schlag unter einem Aufschlagwinkel von weniger als 75°, insbesondere 20° bis 60°, ausübbar ist. Dadurch erfolgt auf konstruktiv einfache Weise eine Übersetzung einer Bewegung der Betätigungseinrichtung, bevorzugt eines linear bewegten Ankers, mit großem Hub und geringer Kraft über einen kleinen Aufschlagwinkel, unter welchem der Schlag ausgeübt wird, auf eine Bewegung der Sperrklinke mit geringem Hub und großer Kraft, sodass eine besonders zuverlässige Auslösung erreicht ist. Dabei gleitet der Anker, mit welchem der Schlag normalerweise ausgeübt wird, über die Sperrklinke, wobei eine Kraftübertragung erfolgt. Hierzu ist der Anker an einem vorderen Ende, mit welchem der Schlag ausgeführt wird, in der Regel konusförmig bzw. mit einer Spitze mit einem dem Aufschlagwinkel entsprechenden Spitzenwinkel ausgebildet, um ein günstiges Gleiten des Ankers über die Sperrklinke bei geringer Flächenpressung zu ermöglichen.

Durch den genannten Aufschlagwinkel wird im Gegensatz zu einer senkrechten Betätigung der Sperrklinke, also eine Betätigung unter einem Aufschlagwinkel von 90°, mit einer entsprechenden Kraft ein kleineres Sicherungssystem mit geringerem benötigten Bauraum erreicht, welches einfach in eine Schusswaffe integrierbar ist. Ein unter einem Aufschlagwinkel von 90° wirkendes Sicherungssystem würde verglichen damit unter anderem einen größeren Energiespeicher zur Abgabe eines höheren Stromes erfordern, um die Sperrklinke mit gleich großer Kraft zu betätigen. Dadurch ist es möglich, die Betätigungseinrichtung und einen Energiespeicher hierzu sowie gegebenenfalls einen Kondensator unsichtbar in einem Schaft oder einem Griffstück der Schusswaffe anzuordnen. Üblicherweise ist die gesamte Betätigungseinrichtung in einem Schaft oder einem Kolben der Schusswaffe angeordnet, sodass diese von außen im Wesentlichen nicht sichtbar ist.

Bevorzugt weist die Betätigungseinrichtung eine Spule zur Erzeugung eines Magnetfeldes sowie einen durch das Magnetfeld bewegbaren Anker auf, mit welchem der Spannschieber betätigbar ist. Eine konstruktive Ausführung der Betätigungseinrichtung mittels eines durch ein Magnetfeld bewegten Ankers hat sich als sehr robuste Ausführung erwiesen. In der Regel wird der Spannschieber indirekt durch den Anker betätigt, wenn der Anker auf eine Einrichtung wirkt, durch welche der Spannschieber in der zweiten Position gehalten wird, beispielsweise eine auf eine drehbare Kulisse wirkende Sperrklinke, welche den Spannschieber in der Regel formschlüssig in der zweiten Position hält. Eine Betätigung des rotatorisch oder translatorisch bewegten Ankers durch das Magnetfeld kann grundsätzlich stoßartig wie bei einem Elektromagneten oder kontinuierlich wie bei einem Motor, insbesondere einem Servomotor, erfolgen.

Es hat sich bewährt, dass eine Rückstellfeder vorgesehen ist, mit welcher der Anker nach einer Betätigung des Spannschiebers in eine Ruheposition bringbar ist. Dies hat sich besonders bei einer Ausbildung der Betätigungseinrichtung als Elektromagnet bewährt, wobei der Anker stabförmig ausgebildet und in der Spule angeordnet ist. Der in der Regel ferromagnetische, mit Vorteil aus Eisen oder Stahl bestehende Anker wird üblicherweise bei einer Aktivierung linear bewegt, in der Regel um einen Stoß auf eine Sperrklinke oder dergleichen auszuführen, sodass ein Entspannen der Schlagfeder bewirkt wird. Es ist dann für eine Herstellung eines sicheren Zustandes der Schusswaffe ein Stromstoß ausreichend, welcher eine lineare Bewegung des Ankers auf die Sperrklinke hervorruft, wodurch der Spannschieber von der Schlagfeder angetrieben in die erste Position bewegt wird. Dadurch ist eine besonders schnelle Auslösung bzw. Entspannung gewährleistet, sodass die Schusswaffe beispielsweise während eines freien Falles in einen sicheren Zustand gebracht werden kann, bevor diese am Boden aufschlägt. Ein Zurückbewegen des Ankers in eine Ausgangsposition in der Spule erfolgt dann ohne elektrische Energiezufuhr durch die Rückstellfeder. Dadurch wird eine robuste Konstruktion erreicht, mit welcher auch bei einer sehr seltenen Betätigung eine sichere Auslösung gewährleistet ist. Normalerweise sind eine Masse des Ankers, eine Federkennlinie der Rückstellfeder sowie ein durch eine Windungsanzahl und eine Stromstärke definiertes Magnetfeld der Spule derart aufeinander abgestimmt, dass eine sichere Auslösung und Rückstellung in jeder möglichen Position der Schusswaffe gewährleistet ist. Dazu ist es günstig, wenn die Spule so ausgelegt ist, dass durch das in der Spule erzeugbare Magnetfeld eine Gewichtskraft des Ankers samt einer Federkraft der Rückstellfeder überwunden werden können. Weiter ist die Rückstellfeder mit Vorteil zur Rückstellung des Ankers auch gegen eine Gewichtskraft des Ankers ausgebildet, um eine Herstellung eines sicheren Zustandes der Schusswaffe und Rückstellung des Ankers auch bei einer auf den Boden fallenden oder verdreht liegenden Schusswaffe zu gewährleisten.

Bevorzugt ist ein mit der Betätigungseinrichtung und dem Energiespeicher verbundener Kondensator zur Aufbringung eines Stromstoßes auf die Betätigungseinrichtung vorgesehen. Dadurch kann bei geringem Bauraum ein zeitlich begrenzter starker Impuls zur Herstellung eines sicheren Zustandes der Schusswaffe abgegeben werden, um eine sichere Auslösung zu gewährleisten. Üblicherweise wird der Kondensator hierzu bei Aktivierung des Sicherungssystems durch den Energiespeicher geladen. Mit Vorteil wird durch einen derartigen Kondensator ein Stromstoß auf eine Spule ausgeübt, in welcher ein unter Einwirkung eines Magnetfeldes linear bzw. translatorisch bewegbarer Anker angeordnet ist, um mit dem Anker eine Sperrklinke oder dergleichen zu betätigen. Durch den mittels des Kondensators hervorgerufenen starken magnetischen Impuls kann auch bei sehr seltener Auslösung der Betätigungseinrichtung eine Losbrechkraft überwunden werden, welche gegebenenfalls zur Bewegung des Ankers nach langer Zeit erforderlich ist. Dadurch ist eine sichere und robuste Auslösung bei einfacher und platzsparender Konstruktion gewährleistet.

Mit Vorteil ist eine Zieloptik vorgesehen, bei welcher eine Beleuchtung durch eine Bewegung des Spannschiebers in die zweite Position aktivierbar ist. Dadurch kann eine Sensorik mehrfach genutzt werden, durch welche eine Position des Spannschiebers erkannt wird. Gegebenenfalls kann die Beleuchtung auch durch den Energiespeicher mit Energie versorgt werden. Neben einer Beleuchtung der Zieloptik können auch weitere Einrichtungen automatisch aktiviert werden, wenn die Schusswaffe entsichert wird, beispielsweise ein mit der Schusswaffe verbundenes Nachtsichtgerät. Weiter kann der Energiespeicher genutzt werden, um einen Entfernungsmesser und gegebenenfalls einen Ballistikrechner mit Energie zu versorgen. Üblicherweise wird durch den Ballistikrechner ein sich aufgrund einer Entfernung ergebender Korrekturwert errechnet, welcher in einer Zieloptik angezeigt wird. Es kann auch vorgesehen sein, dass ein Absehen in einer Zieloptik automatisch aufgrund des errechneten Korrekturwertes um einen entsprechenden Betrag verschoben wird, sodass eine sich aus der Entfernung ergebende Höhenabweichung bereits in der Zieloptik ausgeglichen wird. Auch eine derartige Absehensverstellung kann durch den Energiespeicher mit Energie versorgt werden.

Die weitere Aufgabe wird erfindungsgemäß dadurch gelöst, dass bei einem Verfahren der eingangs genannten Art die Schlagfeder bei Eintritt einer vordefinierten Bedingung durch eine Betätigungseinrichtung automatisch entspannt wird, wobei die Schusswaffe in einen sicheren Zustand gebracht wird. Dadurch wird auf einfache Weise ein robustes Verfahren erreicht, welches eine Auslösung auch nach mehreren Jahren und bei verschiedenen Temperaturen sicherstellt. Üblicherweise erfolgt eine Entspannung der Schlagfeder unter Bewegung des Spannschiebers von der zweiten Position in die erste Position.

Günstig ist es, wenn Bewegungen der Schusswaffe gemessen werden und die Schusswaffe bei einer vordefinierten Beschleunigung und/oder einer vordefinierten Bewegung in einen sicheren Zustand gebracht wird. Dabei sind meist maximale Bewegungen aus der Entsicherungsposition, Beschleunigungen bzw. Beschleunigungsänderungen als Bedingungen definiert, wobei ein Überschreiten derselben eine Herstellung eines sicheren Zustandes auslöst. Somit können Risiken durch Anfänger, welche mit in einem unsicheren Zustand befindlichen Schusswaffen hantieren, einfach vermieden werden.

Zweckmäßigerweise wird das Sicherungssystem durch ein Spannen der Schlagfeder aktiviert. Dies verhindert ein schnelles Entleeren des Energiespeichers, weil bei einer in einem sicheren Zustand befindlichen Schusswaffe im Wesentlichen kein Strom fließt. Üblicherweise werden sämtliche Verbraucher vom Energiespeicher getrennt, wenn die Schusswaffe in einem sicheren Zustand ist, wobei die Schlagfeder entspannt, die Schusswaffe gesichert oder keine Patrone eingelegt ist. In der Regel erfolgt eine Aktivierung des Sicherungssystems durch ein Bewegen des Spannschiebers. Alternativ kann das System auch bei einer Schusswaffe, welche eine Sicherung aufweist, aktiviert werden, wenn die Schusswaffe durch Betätigen der Sicherung aktiv entsichert wird. Es kann jedoch auch eine vorzugsweise elektronische Uhr vorgesehen sein, welche einen geringen Energieverbrauch aufweist und immer mit dem Energiespeicher verbunden ist, um beispielsweise Datum und Uhrzeit einer Schussauslösung protokollieren zu können.

Mit Vorteil wird die Schusswaffe in einen sicheren Zustand gebracht, wenn in einem unsicheren Zustand der Schusswaffe ein Loslassen eines Benutzers erkannt wird. Dadurch wird auf einfache Weise eine Fallerkennung realisiert. Weiter wird somit ein Lagern einer in einem unsicheren Zustand befindlichen Schusswaffe verhindert.

In der Regel wird bei einem Herstellen eines sicheren Zustandes der Schusswaffe durch eine Betätigungseinrichtung ein Schlag auf eine Sperrklinke ausgeübt, welcher ein Entspannen der Schlagfeder auslöst. Weil dadurch eine Mechanik der Schusswaffe zur Sicherung genutzt wird, wird eine einfache Konstruktion erreicht, bei welcher nur die Sperrklinke betätigt werden muss. Eine spezielle Mechanik zur Blockade des Schlagbolzens oder zur gesonderten Entriegelung des Spannschiebers muss dabei nicht vorgesehen sein.

Zweckmäßigerweise wird der Schlag unter einem Aufschlagwinkel von weniger als 75°, insbesondere 20° bis 60°, ausgeübt. Dadurch wird eine Bewegung eines Aktuators, welcher den Schlag ausführt, üblicherweise ein linear bewegter Anker, mit großem Hub und kleiner Kraft auf eine Bewegung der Sperrklinke mit großer Kraft und kleinem Hub übersetzt, sodass eine sichere Auslösung bei geringem Bauraum gewährleistet ist.

Günstig ist es, wenn vor einem Herstellen eines sicheren Zustandes der Schusswaffe ein Warnsignal abgegeben wird. Der Benutzer erhält dadurch die Möglichkeit, die Schusswaffe selbst wieder in einen sicheren Zustand zu bringen. Weiter kann das Warnsignal auch über Funk oder dergleichen an einem mobilen Gerät eines Ausbilders oder weiterer Jäger abgegeben werden, sodass gefährdete Personen von einer unsicheren Schusswaffe informiert werden. Normalerweise wird das Warnsignal optisch, akustisch oder durch Vibrationen abgegeben.

Es hat sich bewährt, dass eine erfindungsgemäß ausgebildete Schusswaffe als Langwaffe, insbesondere als Jagdwaffe eingesetzt wird. Insbesondere zu Ausbildungszwecken bei verschiedensten Witterungen hat sich der Einsatz einer derartigen Schusswaffe als günstig erwiesen, um ein Verletzungsrisiko zu minimieren.

Weitere Merkmale, Vorteile und Wirkungen der Erfindung ergeben sich anhand des nachfolgend dargestellten Ausführungsbeispiels. In den Zeichnungen, auf welche dabei Bezug genommen wird, zeigen:
Fig. 1 eine erfindungsgemäße Schusswaffe;
Fig. 2 eine Betätigungseinrichtung für eine erfindungsgemäße Schusswaffe.

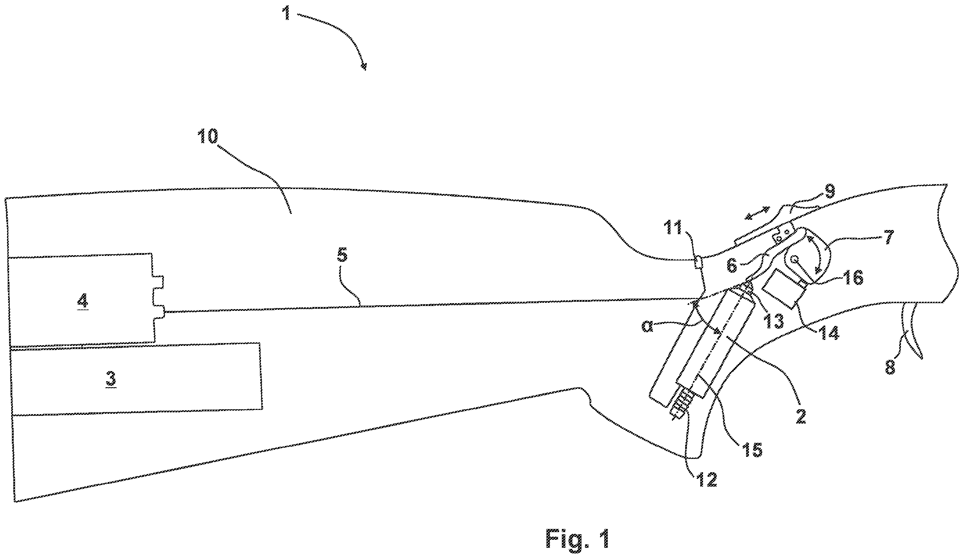

Fig. 1 zeigt eine erfindungsgemäße Schusswaffe 1 in schematischer Darstellung, welche als Jagdgewehr ausgebildet ist. In einem hinteren Teil des Kolbens 10 der Schusswaffe 1 sind ein als Batterie ausgebildeter elektrischer Energiespeicher 3 sowie eine Elektronikeinheit 4 vorgesehen, welche auf eine Betätigungseinrichtung 2 wirken. Um die Schusswaffe 1 in einen schussbereiten bzw. unsicheren Zustand zu bringen, wird ein außenseitiger Spannschieber 9 von einer ersten Position in eine zweite Position bewegt, wobei eine auf einen Schlagbolzen wirkende Schlagfeder gespannt wird. Schlagfeder und Schlagbolzen sind wie ein vorderer Teil der Schusswaffe 1 nicht abgebildet. Zur Abgabe eines Schusses wird der Abzug 8 betätigt, wobei mittels der Schlagfeder durch den Schlagbolzen ein Schlag auf eine Patrone ausgeübt wird, welche eine Treibladung enthält. Nach einem Herstellen eines schussbereiten bzw. unsicheren Zustandes der Schusswaffe 1 und vor Abgabe eines Schusses wird der Spannschieber 9 mittels einer im Kolben 10 der Schusswaffe 1 angeordneten Mechanik in der zweiten Position wie dargestellt gehalten, wobei die Schlagfeder gespannt ist. Hierzu sind eine drehbar in der Schusswaffe 1 angeordnete Kulisse 7 sowie eine Sperrklinke 6 vorgesehen, wobei die Sperrklinke 6 mit der Kulisse 7 verbunden ist und die Kulisse 7 bei einer Bewegung des Spannschiebers 9 von einer ersten Position in eine zweite Position um eine Kulissenachse 16 gedreht wird, um den Spannschieber 9 in der zweiten Position zu halten. Diese Mechanik ist nur schematisch dargestellt.

Um die Schusswaffe 1 automatisch in einen sicheren Zustand zu bringen, ohne dass ein Schuss abgegeben wird, ist die unter der Sperrklinke 6 im Kolben 10 angeordnete Betätigungseinrichtung 2 derart ausgebildet, dass durch einen magnetisch bewegbaren Anker 13 der Betätigungseinrichtung 2 ein Schlag auf die Sperrklinke 6 ausübbar ist. Hierzu ist in der Betätigungseinrichtung 2 eine Spule vorgesehen, um ein auf den Anker 13 wirkendes Magnetfeld zu erzeugen. Weiter ist eine Rückstellfeder 12 vorgesehen, durch welche der Anker 13 nach Ausübung eines Schlages auf die Sperrklinke 6 wieder in eine Ausgangsposition bewegt wird. Bei Ausübung eines Schlages auf die Sperrklinke 6 wird eine Verriegelung des Spannschiebers 9 in der zweiten Position gelöst, wodurch der Spannschieber 9 unter Einwirkung der Schlagfeder wieder in die erste Position bewegt wird. Dadurch wird die Schusswaffe 1 entspannt, und somit in einen sicheren Zustand gebracht. Ein Herstellen eines sicheren Zustandes der Schusswaffe 1 erfolgt somit durch Aufbringung eines Stromstoßes auf die Spule, wodurch ein kurzzeitiges Magnetfeld erzeugt wird, welches eine Bewegung des Ankers 13 hervorruft. Mittels der Elektronikeinheit 4, welche einen Mikroprozessor umfasst, kann eine automatische Herstellung eines sicheren Zustandes der Schusswaffe 1 unter verschiedenen Bedingungen erfolgen. Bevorzugt ist in der Elektronikeinheit 4 eine Einrichtung zur Zeiterfassung vorgesehen, sodass die Schusswaffe 1 automatisch in einen sicheren Zustand gebracht werden kann, wenn sich die Schusswaffe 1 über einen definierten Zeitraum in einem unsicheren Zustand befindet.

Weiter sind mit Vorteil Beschleunigungssensoren in der Elektronikeinheit 4 vorgesehen, durch welche mittels des Mikroprozessors eine Orientierung der Schusswaffe 1 im Raum feststellbar ist. Da die Schusswaffe 1 üblicherweise unmittelbar vor Abgabe eines Schusses in einen unsicheren Zustand gebracht wird, hat es sich bewährt, dass eine automatische Herstellung eines sicheren Zustandes der Schusswaffe 1 dann erfolgt, wenn die Schusswaffe 1 um einen definierten Betrag aus einer Entsicherungsposition bewegt wird, in welcher die Schusswaffe 1 in einen unsicheren Zustand gebracht wurde.

Darüber hinaus ist es günstig, wenn ein Sensor vorgesehen ist, durch welchen ermittelbar ist, ob die Schusswaffe 1 durch einen Benutzer gehalten wird, beispielsweise ein kapazitiver Sensor in einem Griffstück der Schusswaffe 1. Dadurch kann eine automatische Herstellung eines sicheren Zustandes dann erfolgen, wenn die Schusswaffe 1 nicht mehr durch den Benutzer gehalten wird, beispielsweise weil diese fallen gelassen wurde. Es ist somit eine schnelle Herstellung eines sicheren Zustandes der Schusswaffe 1 möglich, bevor die Schusswaffe 1 am Boden aufschlägt, wodurch ein Schuss gelöst werden könnte.

Die dargestellte Schusswaffe 1 weist weiter eine Leuchtdiode 11 auf, durch welche ein unsicherer Zustand der Schusswaffe 1 angezeigt wird, um einem Benutzer die Möglichkeit zu geben, die Schusswaffe 1 entweder manuell zu sichern oder wieder in die Entsicherungsposition zu bringen. Alternativ oder ergänzend können weitere Einrichtungen zur Warnung vorgesehen sein, beispielsweise akustische Warneinrichtungen oder Einrichtungen, welche durch eine Vibration auf einen unsicheren Zustand aufmerksam machen. Darüber hinaus sind in der Elektronikeinheit 4 üblicherweise Mittel zur drahtlosen Kommunikation vorgesehen, sodass beispielsweise auf einem Mobiltelefon oder einem Pager eine Warnung akustisch, optisch oder haptisch erfolgen kann, wenn eine Schusswaffe 1 in einem unsicheren Zustand und eine definierte Warnbedingung erfüllt ist.

Zur Verbindung der Batterie mit der Elektronikeinheit 4, der Betätigungseinrichtung 2 sowie der Leuchtdiode 11 sind schematisch dargestellte Verbindungsleitungen 5 vorgesehen. Um einen Stromverbrauch zu minimieren, ist weiter ein Schalter 14 vorgesehen, durch welchen ein die Elektronikeinheit 4, die Betätigungseinrichtung 2 und den Energiespeicher 3 umfassendes Sicherungssystem nur dann aktiviert wird, wenn die Schusswaffe 1 entsichert wird. Dadurch beginnt das Sicherungssystem erst bei Entsicherung der Schusswaffe 1 mit einer Überprüfung einer Bewegung aus der Entsicherungsposition oder einer Zeitmessung, um die Schusswaffe 1 gegebenenfalls automatisch zu sichern. Ein Entladen der Batterie wird somit auf einfache Weise in einem Zeitraum verhindert, in welchem die Schusswaffe 1 in einem sicheren Zustand ist und eine automatisierte Herstellung eines sicheren Zustandes ohnedies nicht erforderlich ist. Hierzu ist der über die Kulisse 7 bei Bewegung des Spannhebels in die zweite Position betätigte Schalter 14 durch eine nicht dargestellte Leitung mit der Elektronikeinheit 4 verbunden.

Es kann auch eine elektronische Uhr vorgesehen sein, welche nur einen geringen Energieverbrauch aufweist und ständig mit Energie versorgt wird, um beispielsweise Datum und Uhrzeit abgegebener Schüsse protokollieren zu können. Weiter kann ein mit der Elektronikeinheit 4 verbundener Datenspeicher vorgesehen sein, um verschiedene relevante Daten betreffend eine Benutzung der Schusswaffe 1 aufzuzeichnen, um eine optimale Instandhaltung zu gewährleisten.

Die Betätigungseinrichtung 2 wirkt bei der dargestellten Schusswaffe 1 auf die Sperrklinke 6 durch Aufbringung eines Schlages, welcher unter einem Schlagwinkel von weniger als 70°, vorzugsweise 30° bis 60°, insbesondere 40° bis 50° auf die Sperrklinke 6 aufgebracht wird. Durch diesen Schlagwinkel wird eine Bewegung des linear bewegten Ankers 13 mit großem Hub und geringer Kraft in eine Bewegung der Sperrklinke 6 mit großer Kraft und geringem Hub übersetzt, sodass eine zuverlässige Auslösung und Herstellung eines sicheren Zustandes der Schusswaffe 1 gewährleistet sind. Weiter ist üblicherweise ein Kondensator vorgesehen, um einen kurzzeitigen Stromstoß hoher Stromstärke auszulösen, sodass ein Schlag des Ankers 13 mit einer kurzzeitig hohen Kraft erfolgen kann.

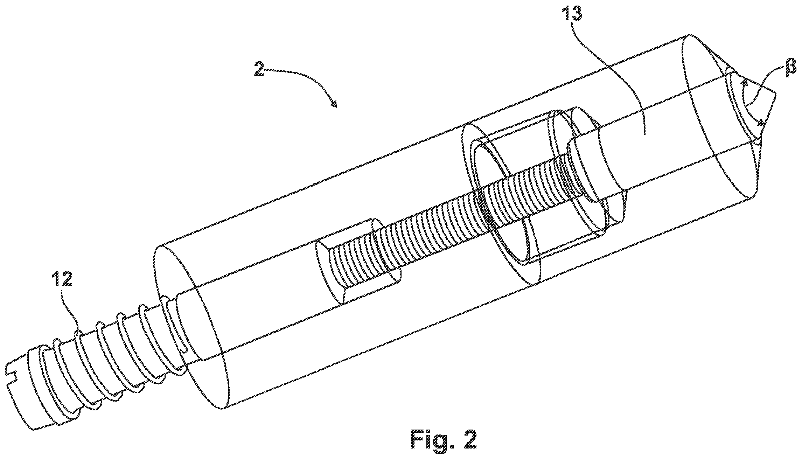

Fig. 2 zeigt eine Betätigungseinrichtung 2 für eine erfindungsgemäße Schusswaffe 1 in schematischer Darstellung. Ersichtlich ist der durch eine nicht dargestellte Spule entlang einer Ankerachse 15 linear bewegbare, etwa zylindrische Anker 13, welcher an einem vorderen Ende mit einer Spitze bzw. als Konus ausgebildet ist. Die Spule ist konzentrisch zum Anker 13 bzw. konzentrisch zur Ankerachse 15 in einem etwa zylindrischen Gehäuse angeordnet. Ein Spitzenwinkel β des Konus ist dabei üblicherweise entsprechend dem Aufschlagwinkel α ausgebildet, unter welchem ein Schlag auf die Sperrklinke 6 ausgeübt wird. Dabei beträgt der Spitzenwinkel β in der Regel das Doppelte des Aufschlagwinkels α, bei der dargestellten Betätigungseinrichtung 2 etwa 90°.

Weiter ist eine Rückstellfeder 12 vorgesehen, durch welche der Anker 13 nach Aufbringung eines Schlages auf die Sperrklinke 6 in eine dargestellte Ausgangsposition bewegt wird. Eine Masse des Ankers 13, eine Kennlinie der Rückstellfeder 12 sowie ein durch eine Windungsanzahl und Stromstärke definiertes Magnetfeld der Spule sind dabei derart aufeinander abgestimmt, dass eine Ausübung des Schlages sowie eine Rückstellung des Ankers 13 in jeder Lage und Position der Schusswaffe 1 möglich sind und keine unerwünschte Auslösung der Betätigungseinrichtung 2 erfolgen kann.

Mit einer erfindungsgemäßen Schusswaffe 1 können Unfälle aufgrund versehentlich nicht in einem sicheren Zustand befindlicher Schusswaffen 1 auf einfache Weise verhindert werden, da unter verschiedensten, variabel definierbaren Bedingungen durch einen Mikroprozessor eine automatische Entspannung der Schusswaffe 1 ausgelöst werden kann. Weiter ist auch ein Verletzungsrisiko aufgrund von umfallenden oder fallengelassenen Schusswaffen 1 auf einfache Weise mittels eines Sensors vermieden, durch welchen eine Berührung der Schusswaffe 1 durch einen Benutzer ermittelbar ist. Darüber hinaus kann auch vorgesehen sein, dass die Schusswaffe 1 automatisch in einen sicheren Zustand gebracht wird, wenn eine Kapazität des Energiespeichers 3 einen definierten Schwellwert unterschreitet, sodass auch bei einer leeren Batterie kein Risiko aufgrund einer ungesicherten Schusswaffe 1 besteht. Alternativ kann bei Unterschreiten eines definierten Schwellwertes auch nur eine Warnung ausgelöst werden, ohne dass die Schusswaffe 1 in einen sicheren Zustand gebracht wird.

Ein übermäßig schnelles Entleeren des Energiespeichers 3 wird durch den Schalter 14 auf einfache Weise verhindert, welcher das Sicherungssystem erst bei Entsicherung der Schusswaffe 1 aktiviert. Neben dem Sicherungssystem können mit dem Schalter 14 auch weitere Zusatzeinrichtungen der Schusswaffe 1 verbunden sein, beispielsweise eine Beleuchtung eines Visiers, eine Absehensverstellung oder eine Zieleinrichtung.

Aufgrund der robusten Ausführung der Betätigungseinrichtung 2, welche einen Schlag zur Entspannung der Schusswaffe 1 auf die Sperrklinke 6 ausübt, kann eine Herstellung eines sicheren Zustandes der Schusswaffe 1 auch unter verschiedensten Temperaturen sowie klimatischen Bedingungen sichergestellt werden. Weiter ist dadurch auch eine zuverlässige Herstellung eines sicheren Zustandes der Schusswaffe 1 nach vielen Jahren gewährleistet.

## Patentansprüche

1. Schusswaffe (1), mit einer auf einen Schlagbolzen wirkenden Schlagfeder, welche durch Bewegen eines Spannschiebers (9) von einer ersten Position in eine zweite Position spannbar ist, um die Schusswaffe (1) in einen schussbereiten Zustand zu bringen, wobei für eine automatische Herstellung eines sicheren Zustandes der Schusswaffe (1) unter vordefinierten Bedingungen ein Sicherungssystem vorgesehen ist, welches einen elektrischen Energiespeicher (3) sowie eine mit dem Energiespeicher (3) verbundene elektromechanische Betätigungseinrichtung (2) umfasst, mittels welcher die Schlagfeder entspannbar ist, wobei das Sicherungssystem zur Herstellung eines sicheren Zustandes der Schusswaffe (1) bei einer vordefinierten Beschleunigung oder einer vordefinierten Bewegung zumindest einen elektrisch wirkenden Beschleunigungssensor in einer Elektronikeinheit aufweist, durch welchen mittels eines Mikroprozessors eine Orientierung der Schusswaffe (1) in einem Raum feststellbar ist, wobei die Betätigungseinrichtung (2) zur Ausführung eines Schlages auf eine den Spannschieber (9) in der zweiten Position fixierende Sperrklinke (6) ausgebildet ist, wobei die Betätigungseinrichtung (2) derart relativ zur Sperrklinke (6) angeordnet ist, dass der Schlag unter einem Aufschlagwinkel (α) von weniger als 75°, insbesondere 20° bis 60°, ausübbar ist.

2. Schusswaffe (1) nach Anspruch 1, **dadurch gekennzeichnet, dass** das Sicherungssystem durch ein Herstellen eines unsicheren Zustandes der Schusswaffe (1) aktivierbar und durch ein Herstellen eines sicheren Zustandes der Schusswaffe (1) deaktivierbar ist.

3. Schusswaffe (1) nach Anspruch 1 oder 2, **dadurch gekennzeichnet, dass** das Sicherungssystem einen Mikroprozessor zur Betätigung der Betätigungseinrichtung (2) umfasst.

4. Schusswaffe (1) nach Anspruch 3, **dadurch gekennzeichnet, dass** ein mit dem Mikroprozessor verbundener Datenspeicher zur Aufzeichnung von schussrelevanten Daten vorgesehen ist.

5. Schusswaffe (1) nach einem der Ansprüche 1 bis 4, **dadurch gekennzeichnet, dass** insbesondere in einem Schaft oder einem Griffstück der Schusswaffe (1) ein Sensor vorgesehen ist, mit welchem eine Berührung der Schusswaffe (1) durch einen Benutzer ermittelbar ist.

6. Verfahren zur Herstellung eines sicheren Zustandes einer Schusswaffe (1) mit einer auf einen Schlagbolzen wirkenden Schlagfeder, welche spannbar ist, insbesondere einer Schusswaffe (1) nach einem der Ansprüche 1 bis 5, wobei die Schlagfeder bei Eintritt einer vordefinierten Bedingung durch eine Betätigungseinrichtung (2) automatisch entspannt wird, wobei die Schusswaffe (1) in einen sicheren Zustand gebracht wird, wobei Bewegungen der Schusswaffe (1) mit zumindest einem elektrisch wirkenden Beschleunigungssensor gemessen werden und die Schusswaffe (1) bei einer vordefinierten Beschleunigung und/oder einer vordefinierten Beschleunigungsänderung in einen sicheren Zustand gebracht wird, wobei bei einem Herstellen eines sicheren Zustandes der Schusswaffe (1) durch eine Betätigungseinrichtung (2) ein Schlag auf eine Sperrklinke (6) ausgeübt wird, welcher ein Entspannen der Schlagfeder auslöst, wobei der Schlag unter einem Winkel von weniger als 75°, insbesondere 20° bis 60°, ausgeübt wird.

7. Verfahren nach Anspruch 6, **dadurch gekennzeichnet, dass** das Sicherungssystem durch ein Spannen der Schlagfeder aktiviert wird.

8. Verfahren nach Anspruch 6 oder 7, **dadurch gekennzeichnet, dass** die Schusswaffe (1) in einen sicheren Zustand gebracht wird, wenn in einem unsicheren Zustand der Schusswaffe (1) ein Loslassen eines Benutzers erkannt wird.

9. Verfahren nach einem der Ansprüche 6 bis 8, **dadurch gekennzeichnet, dass** vor einem Herstellen eines sicheren Zustandes der Schusswaffe (1) ein Warnsignal abgegeben wird.

10. Verwendung einer Schusswaffe (1) nach einem der Ansprüche 1 bis 5 als Langwaffe, insbesondere als Jagdwaffe.

## Claims

1. A firearm (1) with a main spring that acts upon a striking pin and can be tensioned from a first position into a second position by moving a cocking slide (9) in order to transfer the firearm (1) into a ready-to-fire state, wherein a safety system is provided for automatically establishing a safe state of the firearm (1) under predefined conditions, wherein said safety system comprises an electric energy storage (3), as well as an electromechanical actuating device (2) that is connected to the energy storage (3) and makes it possible to release the tension of the main spring, wherein the safety system features at least one electric acceleration sensor in an electronics unit in order to establish a safe state of the firearm (1) when a predefined acceleration or a predefined motion occurs and said the acceleration sensor makes it possible to determine an orientation of the firearm (1) in space by means of a microprocessor, wherein the actuating device (2) is designed for striking a pawl (6), which fixes the cocking slide (9) in the second position, and wherein the actuating device (2) is arranged relative to the pawl (6) in such a way that the strike can take place at an impact angle (α) of less than 75°, particularly 20° to 60°.

2. The firearm (1) according to claim 1, **characterized in that** the safety system can be activated by establishing an unsafe state of the firearm (1) and deactivated by establishing a safe state of the firearm (1).

3. The firearm (1) according to claim 1 or 2, **characterized in that** the safety system comprises a microprocessor for actuating the actuating device (2).

4. The firearm (1) according to claim 3, **characterized in that** a data memory for recording shot-relevant data is provided and connected to the microprocessor.

5. The firearm (1) according to one of claims 1-4, **characterized in that** a sensor is provided, in particular, in a stock or a grip of the firearm (1) and makes it possible to determine whether the firearm (1) is held by a user.

6. A method for establishing a safe state of a firearm (1) with a main spring that acts upon a striking pin and can be tensioned, particularly a firearm (1) according to one of claims 1-5, wherein the tension of the main spring is automatically released by an actuating device (2) when a predefined condition occurs and the firearm (1) is thereby transferred into a safe state, wherein motions of the firearm (1) are measured with at least one electric acceleration sensor and the firearm (1) is transferred into a safe state when a predefined acceleration and/or a predefined acceleration change occurs, wherein an actuating device (2) strikes a pawl (6), which triggers a release of the tension of the main spring, when a safe state of the firearm (1) is established, and wherein the strike takes place at an angle of less than 75°, particularly 20° to 60°.

7. The method according to claim 6, **characterized in that** the safety system is activated by tensioning the main spring.

8. The method according to claim 6 or 7, **characterized in that** the firearm (1) is transferred into a safe state when it is detected that a user lets go of the firearm (1) in an unsafe state thereof.

9. The method according to one of claims 6-8, **characterized in that** a warning signal is emitted prior to establishing a safe state of the firearm (1).

10. The utilization of a firearm (1) according to one of claims 1-5 as a long firearm, particularly a hunting rifle.

## Revendications

1. Arme à feu (1) avec un ressort de percussion agissant sur un percuteur, lequel peut être tendu par déplacement d'un levier d'armement (9) d'une première position dans une deuxième position afin de mettre l'arme à feu (1) dans un état prêt à tirer, pour un établissement automatique d'un état sécurisé de l'arme à feu (1) dans des conditions prédéfinies, un système de sécurité étant prévu, lequel comprend un accumulateur d'énergie électrique (3) ainsi qu'un dispositif d'actionnement électromécanique (2) relié à l'accumulateur d'énergie (3), au moyen duquel le ressort de percussion peut être détendu, le système de sécurité comportant pour l'établissement d'un état sécurisé de l'arme à feu (1) à une accélération prédéfinie ou un déplacement prédéfini, au moins un capteur d'accélération agissant électriquement dans une unité électronique par lequel une orientation de l'arme à feu (1) peut être constatée dans un espace au moyen d'un microprocesseur, le dispositif de commande (2) étant constitué pour exécuter une percussion sur un cliquet de retenue (6) fixant le levier d'armement (9) dans la deuxième position, le dispositif d'actionnement (2) étant disposé par rapport au cliquet de retenue (6) de telle sorte que la percussion peut être pratiquée sous un angle d'impact (α) de moins de 75°, notamment 20° à 60°.

2. Arme à feu (1) selon la revendication 1, **caractérisée en ce que** le système de sécurité peut être activé par établissement d'un état non sécurisé de l'arme à feu (1) et être désactivé par établissement d'un état sécurisé de l'arme à feu (1).

3. Arme à feu (1) selon la revendication 1 ou 2, **caractérisée en ce que** le système de sécurité comprend un microprocesseur pour actionner le dispositif d'actionnement (2).

4. Arme à feu (1) selon la revendication 3, **caractérisée en ce qu'**une mémoire de données reliée au microprocesseur est prévue pour enregistrer des données relatives au tir.

5. Arme à feu (1) selon l'une quelconque des revendications 1 à 4, **caractérisée en ce que** notamment dans une crosse ou une poignée de l'arme à feu (1) est prévu un capteur avec lequel un contact de l'arme à feu (1) par un utilisateur peut être déterminé.

6. Procédé pour établir un état sécurisé d'une arme à feu (1) avec un ressort de percussion agissant sur un percuteur, lequel peut être tendu, notamment d'une arme à feu (1) selon l'une quelconque des revendications 1 à 5, le ressort de percussion étant automatiquement détendu par un dispositif d'actionnement (2) à l'introduction d'une condition prédéfinie, l'arme à feu (1) étant mise dans un état sécurisé, les mouvements de l'arme à feu (1) étant mesurés avec au moins un capteur d'accélération agissant électriquement et l'arme à feu (1) étant mise dans un état sécurisé lors d'une accélération prédéfinie et/ou d'une modification d'accélération prédéfinie, lors de l'établissement d'un état sécurisé de l'arme à feu (1) par un dispositif d'actionnement (2), une percussion sur un cliquet de retenue (6) étant exercée, laquelle déclenche une détente du ressort de percussion, la percussion étant exercée sous un angle de moins de 75°, notamment 20° à 60°.

7. Procédé selon la revendication 6, **caractérisé en ce que** le système de sécurité est activé par une tension du ressort de percussion.

8. Procédé selon la revendication 6 ou 7, **caractérisé en ce que** l'arme à feu (1) est mise dans un état sécurisé, si dans un état non sécurisé de l'arme à feu (1), un relâchement de l'utilisateur est identifié.

9. Procédé selon l'une quelconque des revendications 6 à 8, **caractérisé en ce qu'**un signal d'alerte est émis avant établissement d'un état sécurisé de l'arme à feu (1).

10. Utilisation d'une arme à feu (1) selon l'une quelconque des revendications 1 à 5 en tant qu'arme longue, notamment en tant qu'arme de chasse.
